# EUROPÄISCHE PATENTSCHRIFT

(11) **EP 2 500 059 B1**
(45) Veröffentlichungstag und Bekanntmachung des Hinweises auf die Patenterteilung: **08.01.2014**
(21) Anmeldenummer: 12156462.9
(22) Anmeldetag: 22.02.2012
(51) Int. Cl.: A61N 1/05

(54) **Symmetrische Kontaktfeder für Schraubköpfe**
Symmetrical contact spring for screw heads
Ressort de contact symétrique pour têtes de vis

(30) Priorität: 17.03.2011 DE 102011001360
(43) Veröffentlichungstag der Anmeldung: 19.09.2012
(73) Patentinhaber: BIOTRONIK SE & Co. KG, 12359 Berlin (DE)
(72) Erfinder: Kolberg, Gernot, 12161 Berlin (DE); Schilk, Carsten, 12623 Berlin (DE)
(74) Vertreter: Lindner-Vogt, Karin L.

(56) Entgegenhaltungen:
- EP-A1- 1 762 266
- DE-A1-102004 035 987
- DE-B3-102004 002 404
- US-A- 6 018 683
- US-B1- 6 687 550

## Beschreibung

Gegenstand der Patentanmeldung ist eine Elektrodenleitung zur Implantation in einen lebenden Körper sowie eine Kontaktfeder zur elektrischen Kontaktierung zwischen einem außen liegenden Gehäuse, beispielsweise ein Elektrodenkopfgehäuse, und einer innen liegenden Wendel, die beispielsweise zur aktiven Befestigung der Elektrodenleitung dient.

Eine Elektrodenleitung der eingangs genannten Art umfasst einen lang gestreckten Elektrodenkörper zumeist aus einem elektrisch isolierenden Material, eine im Elektrodenkörper verlaufende, drehbare, elektrische Zuleitung und einen Elektrodenkopf am distalen Ende des Elektrodenkörpers. Der Elektrodenkopf umfasst dabei unter anderem ein Gehäuse, eine darin mit Hilfe der Zuleitung drehbar und axial verschiebbar gelagerte, elektrisch leitfähige Welle, die an der Zuleitung in axialer Verlängerung angesetzt ist, eine korkenzieherartige Einschraubelektrode an der Welle, sowie eine zwischen Gehäuse und Welle eingesetzte Kontaktfeder zu deren elektrischer Verbindung.

Verschiedene Kontaktfedern sind bereits im Markt etabliert. Gemäß einer Ausführung ist sie eine Schraubendruckfeder. Sie baut die Kontaktkraft in axialer Richtung zwischen einem Kontaktlager an der Fixierungsachse und einem Gegenlager im Gehäuse auf. Von besonderem Nachteil dieser Konstruktion ist, dass die Feder den gesamten Schraubbereich der Fixierung abdeckt, also üblicherweise circa 2 mm. Die Feder stellt einen elektrischen Kontakt in allen Positionen her. Ebenso muss die Länge der gestauchten Feder zu der Länge des Kopfes addiert werden muss. Wenn der Kopf zusätzlich eine Dichtung erhält, muss auch deren Länge hinzuaddiert werden. Ein Elektrodenkopf mit dieser Art Feder ist somit für viele Anwendungen zu lang.

In einer anderen Version wird die Kontaktkraft radial zwischen Welle und Gehäuse aufgebaut, was beispielsweise aus der US 2007/0055335 A1 bekannt ist. Dort wird eine Spiralfeder zur elektrischen Verbindung zwischen Elektrodengehäuse und Welle offenbart, die als eine die Welle unter Federspannung umlaufende, im wesentlichen ebene Spiralfeder ausgebildet ist und mit ihren Schenkelenden unter Ausübung einer radial gerichteten Federkraft gleitend mit dem Gehäuse und/oder der Welle in Kontakt steht. Eine Kontaktfeder dieser Bauart hat die Problematik, dass die radiale Kontaktkraft einseitig auf die Welle der Fixierung wirkt. Die Welle benötigt ein radiales Gegenlager, an dem Reibung entstehen kann. Diese Reibung erhöht die Ausschraubzahlen der Fixierung aus dem Elektrodenkopf.

Aufgabe ist es daher, die Probleme des genannten Standes der Technik zu vermeiden und eine Kontaktfeder, welche eine möglichst geringe axialer Erstreckung aufweist und in der die einseitigen radialen Kräfte nicht auftreten oder zumindest kompensiert sind, sowie eine Elektrodenleitung zu schaffen, welche einen möglichst kleinen Elektrodenkopf und einen möglichst langen flexiblen Abschnitt aufweist.

Die Aufgabe wird mit den Ansprüchen 1 bzw. Anspruch 14 gelöst.

Das Dokument US-B-6 687 550 offenbart den nächstliegenden Stand der Technik.

Eine eine Außenkontur und eine Mittelachse aufweisende Kontaktfeder zur elektrischen Kontaktierung eines außen liegenden Gehäuses, beispielsweise eines Elektrodenkopfgehäuses mit einer innerhalb des Gehäuses gelagerten Welle zeichnet sich dadurch aus, dass der Draht der Kontaktfeder so geformt ist, dass er innerhalb der Außenkontur mindestens zwei Schenkel bildet, wobei die Lage der mindestens zwei Schenkel im kontaktierten Zustand der Kontaktfeder durch Krafteinwirkung ausgehend vom Gehäuse oder von der Welle von einer vorbestimmten Lage im nicht kontaktierten Zustand entfernt ist.

Die beiden Schenkel dienen dazu, eine Welle gleitend und axial verschiebbar aufzunehmen bzw. zu lagern. Im kontaktierten Zustand ergibt sich eine Lageänderung gegenüber der vorbestimmten Lage der Schenkel bezüglich der Außenkontur der Kontaktfeder im nicht kontaktierten Zustand. Dabei ändern sich die Spannungsverhältnisse innerhalb der Kontaktfeder gegenüber dem nicht kontaktierten Zustand, welche durch Kräfte - hervorgerufen beispielsweise durch Verschieben der Schenkel aus ihrer vorbestimmten Lage oder durch Kompression der Außenkontur- entstehen. Diese Änderung der Spannungsverhältnisse bewirkt eine elektrische Kontaktierung zwischen jedem Schenkel und der Welle einerseits und zwischen Außenkontur und Gehäuse andererseits.

In einer ersten Ausführungsform liegt jeder der beiden Schenkel im nicht kontaktierten Zustand lateral versetzt zur Mittelachse der Kontaktfeder. Im kontaktierten Zustand der Kontaktfeder liegen die Schenkel von ihrer vorherigen Lage verschoben weitgehend fluchtend zur Mittelachse. Diese Lage wird dadurch erreicht, dass die Schenkel von ihrer vorbestimmten Lage durch Krafteinwirkung auf die Schenkel erfolgt, Sie entsteht beispielsweise dadurch, dass die Schenkel durch eine durch die Mittelachse der Kontaktfeder verlaufende Welle ausgerichtet sind. Bevorzugt weisen sie denselben Abstand zur Mittelachse auf und liegen weiter bevorzugt diametral bezüglich der Mittelachse gegenüber. Durch die so vorhandene Symmetrie entstehen beim soeben genannten Vorspannen im Betrag gleich große Kräfte, die sich jedoch an der Welle aufgrund der diametralen Ausrichtung der Mittelpunktachsen der Schenkel gegenseitig aufheben. So wird eine einseitige Belastung vermieden, was die genannten Nachteile des Standes der Technik beseitigt. Zudem wird die Zuverlässigkeit der Kontaktierung zwischen der Welle mit der schraubendruckfederartigen elektrischen Fixierung und dem Elektrodenkopfgehäuse verbessert, da die Kontaktierung redundant vorliegt.

Handelt es sich bei den beiden Schenkeln bevorzugt um Ösen, weisen diese einen Mittelpunkt auf. Die Mittelpunkte dieser beiden Ösen liegen je auf einer Mittelpunktachse, wobei diese Achsen parallel zur Mittelachse der Kontaktfeder verlaufen. Die Außenkontur der Kontaktfeder entspricht dabei in etwa der Innenkontur des hohlzylinderartigen Elektrodenkopfgehäuses. Werden nun die beiden lateral, vorzugsweise diametral versetzten Ösen durch eine Auslenkungsbewegung (bedingt durch das Einfädeln der Welle) zentriert, erweitert sich die Außenkontur der Kontaktfeder im Durchmesser so, dass der Durchmesser dann größer ist als die Innenkontur des Elektrodenkopfgehäuses. Jedoch kann sich der Außendurchmesser durch die räumliche Begrenzung wegen der unflexiblen Innenkontur des Gehäuses nicht ausweiten, wodurch dann eine Verspannung an den Ösen erfolgt, die sich in einer elektrischen Kontaktierung der Ösen mit der Welle und dem Gehäuse widerspiegelt.

Genauso ist in einer alternativen zweiten Ausführung denkbar, dass die Kontaktfeder so gefertigt ist, dass die Schenkel im nicht kontaktierten Zustand nicht lateral zur Mittelachse versetzt sind, also in der Mittelachse der Kontaktfeder fluchtend liegen. Auch in dieser Ausführung ist die Welle in den Schenkeln gleitend und axial verschiebbar gelagert. Jedoch wird in diesem Fall der kontaktierte Zustand durch eine Veränderung des Durchmessers der Außenkontur erreicht, indem er durch in Richtung Mittelachse weisende Krafteinwirkung verkleinert ist. Die Außenkontur ist also im kontaktierten Zustand gegenüber dem nicht kontaktierten Zustand komprimiert. Dazu ist es notwendig, dass der Durchmesser der Außenkontur im entspannten, nicht kontaktierten Zustand größer als die Innenkontur des außen liegenden Gehäuses ist, welches mit der Welle kontaktiert werden soll. Beim Einsetzen der Kontaktfeder in das Gehäuse wird diese Außenkontur radial nach innen gepresst und in das Gehäuse eingeführt. Dabei stellt sich der kontaktierte Zustand ein, da die Schenkel aus der ursprünglichen, auf der Mittelachse fluchtenden Position entfernt sind, indem sie radial nach außen geschoben werden. Dadurch ergibt sich ein elektrischer Kontakt zur Welle. Auch hier kann es sich bei der Ausgestaltung der Schenkel um Ösen handeln.

Die Kontaktfeder ist in beiden Ausführungen bevorzugt als Schenkelfeder - also eine Schraubenfeder, die um ihre Achse auf Torsion beansprucht wird - aufgebaut, welche mit ihrem Außendurchmesser die Außenkontur der Kontaktfeder bildet. Da die axiale Erstreckung des Elektrodenkopfes hauptsächlich von der Kontaktfeder abhängt, muss die axiale Erstreckung letzterer gering gehalten werden, in der Regel durch eine Höhe von 0,1 mm bis 5 mm, bevorzugt 0,5 mm, um einen möglichst kleinen Elektrodenkopf zu erhalten. Um die Zuverlässigkeit zu erhöhen, besteht diese Schraubfeder aus vier bis zehn Drahtwicklungen, bevorzugt vier bis sechs, besonders bevorzugt vier Drahtwicklungen, die sich um die Mittelachse der Kontaktfeder herum erstrecken. Eine Drahtwicklung ist im Sinne der Patentanmeldung der Verlauf des Drahtes mit einem Umschlingungswinkel von 360°, wobei die Steigung dabei dem einfachen Drahtdurchmesser entspricht. Durch die vier Wicklungen ist eine ausreichende Kontaktierung zwischen Kontaktfeder und Elektrodenkopfgehäuse gewährleistet. Weiter bevorzugt weist die Außenkontur einen Querschnitt auf, der mehreckig, vorzugsweise dreieckig, oder gerundet, vorzugsweise kreisrund, oval oder gleichdick ist.

In beiden Ausführungen der Kontaktfeder sind bevorzugt maximal zwei der vier Drahtwicklungen der Kontaktfeder vom Außendurchmesser radial nach innen geformt oder gebogen und bilden die genannten Schenkel, wobei diese maximal zwei Wicklungen in ihrem Durchmesser kleiner sind als der Außendurchmesser. Bevorzugt sind die Durchmesser dieser zwei Wicklungen so dimensioniert, dass die Welle innerhalb des Elektrodengehäuses der genannten Art dreh- und axial verschiebbar gelagert ist. Bevorzugt handelt es sich dabei um die beiden mittleren Wicklungen. So ist gewährleistet, dass durch das unmittelbare nebeneinander Liegen der Ösen die Kraftansetzpunkte bei Verspannungen axial nahe beieinander liegen, so dass sicher ist, dass sich die beiden diametral erstreckenden Kraftvektoren weitgehend kompensieren. Bei der hier genannten Ausführung ist durch die beiden Wicklungen, die sich noch im Außendurchmesser der Schenkelfeder und damit auf der Außenkontur der Kontaktfeder befinden, der elektrische Kontakt zum Gehäuse ebenfalls gewährleistet.

Im Übrigen kann die Kontaktfeder aus jeder Art von Draht hergestellt sein, vorzugsweise ist der Draht ein Rund- oder Flachdraht. Als Material bietet sich insbesondere MP35N oder medizinischer Edelstahl wie Chrom-Kobalt-Legierungen an, aber auch Titan, Tantal, Platin oder Legierungen aus mehreren oder allen dieser drei Elemente. Denkbar sind auch beschichtete Drähte, so genannte "plated wires".

Die in den vorangehenden Ausführungen schon mehrfach erwähnte Elektrodenleitung zur kardiologischen Anwendung umfasst dabei:
- einen lang gestreckten Elektrodenkörper aus einem elektrisch isolierenden Material,
- eine im Elektrodenkörper verlaufende, drehbar gelagerte, elektrische Zuleitung, und
- einen Elektrodenkopf am distalen Ende des Elektrodenkörpers mit
- einem hohlzylinderartigem Elektrodenkopfgehäuse,
- einer im Elektrodenkopfgehäuse mit Hilfe der Zuleitung drehbar und axial verschiebbar gelagerten, elektrisch leitfähigen Welle, die an der Zuleitung in axialer Verlängerung befestigt ist,
- einer schraubendruckfederartigen, starren Einschraubelektrode an der Welle, sowie
- einer zwischen Gehäuse und Welle eingesetzten Kontaktfeder gemäß des soeben beschriebenen Gegenstandes der Patentanmeldung zum Zwecke der elektrischen Verbindung zwischen Gehäuse und Welle, wobei die Kontaktfeder einen Außendurchmesser aufweist, der mit dem Gehäuse elektrisch leitend kontaktierbar ist,

Die Elektrodenleitung zeichnet sich dadurch aus, dass die Welle zwischen zwei Schenkeln der Kontaktfeder gleitend und axial verschiebbar gelagert ist und dass durch die in die elektrische Kontaktierung verbrachte Kontaktfeder radial nach außen gerichtete Federkräfte auf die Schenkel wirken, so dass dadurch die Lage der Schenkel von der vorbestimmten Lage im nicht kontaktierten Zustand entfernt ist und dadurch ein elektrischer Kontakt zwischen Gehäuse und Welle entsteht. Im eingebauten Zustand ist also das innere Gefüge bzw. die Form der Kontaktfeder so verändert, dass dadurch Verspannung entstehen, die zu einer weiter oben beschriebenen Verschiebung der Schenkel bezüglich ihrer ursprünglichen Lage im nicht kontaktierten Zustand führt. Vorzugsweise sind die radial nach außen wirkenden Kräfte in ihrem Betrag im Wesentlichen identisch, wirken aber in diametral entgegen gesetzte Richtung. Dadurch wird die Welle von einseitiger Belastung befreit.

Zur Sicherstellung des im Wesentlichen gleich großen Kraftansatzes weist das Gehäuse ein Wellenlager auf, das die Welle auf einer Gehäusemittelachse lagert, wodurch sich die radial nach außen gerichteten Federkräfte gegenseitig kompensieren.

Bei der Elektrodenleitung handelt es sich um eine so genannte aktiv fixierbare Elektrodenleitung. Das heißt, dass die Elektrode mittels der schraubendruckfederartigen (korkenzieherartige) Einschraubelektrode im Gewebe befestigt werden kann. Um die Einschraubbewegung durchzuführen, kann der Arzt bei der Implantation die elektrische Zuleitung, die auch in axialer Richtung dehnbar sein kann, um deren Achse drehen und so die Einschraubelektrode in Drehung versetzen. Dazu umfasst die Elektrodenleitung am gegenüberliegenden proximalen Ende einen drehbaren Stecker oder drehbaren Steckerpin.

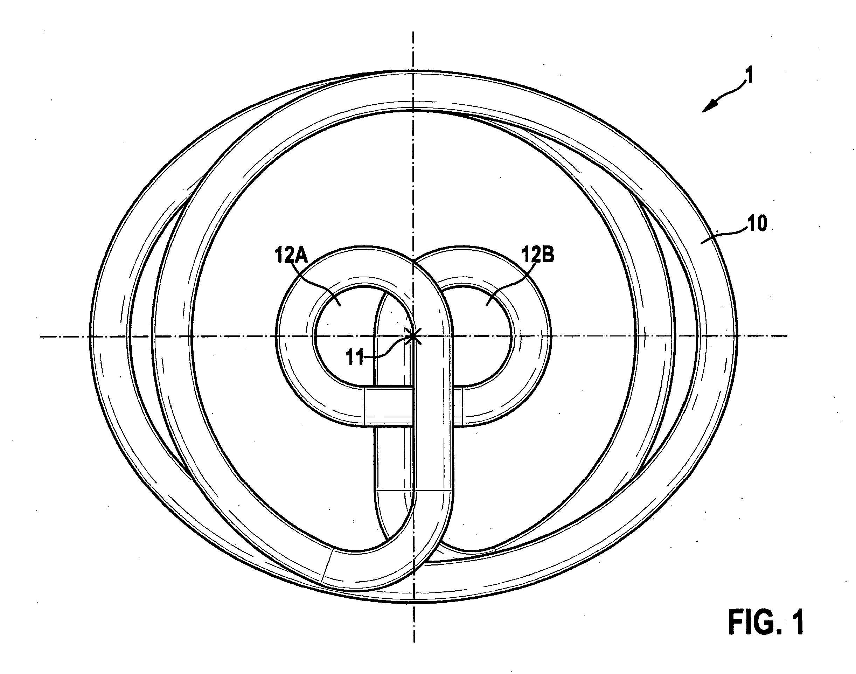

FIG.1 zeigt eine Kontaktfeder gemäß der ersten Ausführung des Gegenstandes dieser Patentanmeldung. Die Kontaktfeder 1 dieses Ausführungsbeispiels ist aus einer aus einem Draht geformten Schenkelfeder 10 geformt, welche einen Außendurchmesser mit einer Mittelachse11 aufweist. Der Außendurchmesser ist so gestaltet, dass er in einem Gehäuse eines hier nicht dargestellten Elektrodenkopfes eingeführt und elektrisch verbunden werden kann. Die Kontaktfeder wird dabei so in das hohlzylindrische Gehäuse des Elektrodenkopfs eingelegt, dass die Mittelachse auf der Längserstreckungsachse des hohlzylindrischen Gehäuses liegt. Der Draht ist dabei so geformt, dass innerhalb des Außendurchmessers zwei Ösen 12A und 12B entstehen, die jeweils einen Mittelpunkt aufweisen. Die Mittelpunkte der Ösen befinden sich je auf einer Achse, die sich parallel zur Mittelachse 11 erstrecken. Die Ösen dienen zur Aufnahme einer hier ebenfalls nicht dargestellten Welle, die im Elektrodengehäuse gelagert ist, wobei das Wellenlager so positioniert ist, dass sich die Welle in der Regel in der Nähe oder auf der Längserstreckungsachse des hohlzylindrischen Elektrodenkopfes und damit auch auf der Mittelachse der Kontaktfeder befindet. Die Mittelpunkte der Ösen 12A und 12B befinden sich im Ruhezustand nicht auf dem der Mittelachse der Kontaktfeder, sondern sind lateral beidseits der Mittelachse versetzt. Bevorzugt ist dabei der Abstand zwischen jeder der Mittelpunktachsen der Ösen und der Mittelachse gleich groß. Erst durch die Einfädelung der Welle in die Ösen wird die Kontaktfeder vorgespannt, das heißt, die Mittelpunktachsen der Ösen 12A und 12 B werden durch die Welle der Elektrodenleitung auf die Mittelachse der Kontaktfeder verlagert und dort gehalten.

Im gezeigten Ausführungsbeispiel besteht die Schenkelfeder aus vier Wicklungen, wobei die beiden äußeren Wicklungen mit dem kompletten Umschlingungswinkel den Außendurchmesser der Schenkelfeder und damit die Außenkontur der Kontaktfeder bilden, während die beiden inneren Wicklungen vom Außendurchmesser radial nach innen geformt oder gebogen sind und dabei die Ösen 12A und 12B bilden. Lediglich die beiden inneren Wicklungen werden bei der Einfädelung der Welle einer Verspannung ausgesetzt und führen auch nur dort zu einer Verformung, während die beiden äußeren Wicklungen aufgrund der Verformung der inneren Wicklungen gegen die Innenkontur des Elektrodenkopfgehäuses gedrückt werden. Die Verformung entsteht durch die oben beschriebenen radial diametral nach außen wirkenden Kräfte. Diese Kräfte sorgen dafür, dass die Ösen ständig in elektrischen Kontakt mit der Welle stehen, die in diesen Ösen gleitend und drehend gelagert ist.

Die Kontaktfeder wird im entspannten Ruhezustand in das Gehäuse eingelegt. Anschließend werden die Ösen so bewegt, dass deren Mittelpunktachsen auf der Mittelachse der Kontaktfeder liegen. Anschließend wird die Welle entlang der Mittelpunktachse durch die beiden Ösen und so in das Wellenlager geführt, dass sie sich entlang Längserstreckungsachse des Gehäuses befindet. Wenn die Welle die Ösen in den Kontaktfedermittelachse zieht, bauen die jeweils der Öse gegenüber liegenden Radien der Kontaktfeder eine Kraft gegen das Gehäuse auf. Die an der Welle angreifenden Kräfte sind symmetrisch und kompensieren sich. Die Welle wird durch die Feder gleitend und axial verschiebbar gelagert. Gleichzeitig entsteht eine Kontaktkraft zwischen Gehäusewand und Kontaktfeder. Der elektrische Kontakt ist zuverlässig gesichert. Auch bei Auslenken der Welle bleibt der Kontakt erhalten, weil der Kontakt immer auf einer Seite anzieht. Dadurch wird das Ein- und Ausschraubverhalten bei Schraubelektroden deutlich verbessert.

## Patentansprüche

1. Kontaktfeder (1) zur elektrischen Kontaktierung eines Elektrodenkopfgehäuses mit einer innerhalb des Elektrodenkopfgehäuses gelagerten Welle, wobei die Kontaktfeder eine Außenkontur mit einer Mittelachse (11) aufweist, wobei die Außenkontur mit dem Gehäuse elektrisch leitend kontaktierbar ist, wobei der Draht der Kontaktfeder so geformt ist, dass er innerhalb der Außenkontur mindestens zwei Schenkel bildet, wobei die Lage der mindestens zwei Schenkel im kontaktierten Zustand der Kontaktfeder durch Krafteinwirkung von einer vorbestimmten Lage im nicht kontaktireten Zustand entfernt ist, **dadurch gekennzeichnet, dass** die Außenkontur der Kontaktfeder aus vier bis zehn Drahtwicklungen gebildet ist.

2. Kontaktfeder nach Anspruch 1, **dadurch gekennzeichnet, dass** deren Außenkontur aus vier bis sechs, bevorzugt vier Drahtwicklungen gebildet ist.

3. Kontaktfeder nach Anspruch 1 oder 2, **dadurch gekennzeichnet, dass** die Drahtwicklungen so um die Mittelachse herum gewickelt sind, dass die Außenkontur einen Querschnitt aufweist, der mehreckig, vorzugsweise dreieckig, oder gerundet, vorzugsweise kreisrund, oval oder gleichdick ist.

4. Kontaktfeder nach Anspruch 1 bis 3, **dadurch gekennzeichnet, dass** maximal zwei der Drahtwicklungen vom Außendurchmesser radial nach innen geformt oder gebogen sind, wobei diese maximal zwei Wicklungen in ihrem Durchmesser kleiner sind als der Außendurchmesser.

5. Kontaktfeder nach Anspruch 4, **dadurch gekennzeichnet, dass** die zwei radial nach innen geformten oder gebogenen Drahtwicklungen die zwei Schenkel bilden.

6. Kontaktfeder nach Anspruch 5, **dadurch gekennzeichnet, dass**, die zwei Schenkel je eine Öse bilden.

7. Kontaktfeder nach einem der Ansprüche 1 bis 6, **dadurch gekennzeichnet, dass** die Schenkel im nicht kontaktierten Zustand lateral versetzt zur Mittelpunktachse der Kontaktfeder und im kontaktierten Zustand durch Krafteinwirkung auf die Schenkel von ihrer vorbestimmten Lage verschoben fluchtend zur Mittelachse liegen.

8. Kontaktfeder nach Anspruch 7, **dadurch gekennzeichnet, dass** die Schenkel je denselben Abstand zur Mittelachse der Kontaktfeder aufweisen.

9. Kontaktfeder nach Anspruch 7 oder 8, **dadurch gekennzeichnet, dass** jeder der Schenkel diametral gegenüber bezüglich der Mittelachse der Kontaktfeder liegt.

10. Kontaktfeder nach einem der Ansprüche 1 bis 6, **dadurch gekennzeichnet, dass** die Schenkel im nicht kontaktierten Zustand fluchtend zur Mittelachse liegen und der kontaktierte Zustand durch in Richtung Mittelachse weisende Krafteinwirkung auf die Außenkontur erreicht wird, wodurch die Schenkel von der vorbestimmten Lage entfernt sind.

11. Kontaktfeder nach einem der Ansprüche 1 bis 10, **dadurch gekennzeichnet, dass** deren Höhe 0,5 mm bis 5 mm, vorzugsweise 0,5 mm ist.

12. Kontaktfeder nach einem der Ansprüche 1 bis 11, **dadurch gekennzeichnet, dass** der Draht ein Rund- oder Flachdraht ist.

13. Kontaktfeder nach einem der Ansprüche 1 bis 12, **dadurch gekennzeichnet, dass** sie als Schenkelfeder aufgebaut ist.

14. Elektrodenleitung zur kardiologischen Anwendung umfassend
- einen langgestreckten Elektrodenkörper aus einem elektrisch isolierenden Material,
- eine im Elektrodenkörper verlaufende, drehbare, elektrische Zuleitung, und
- einen Elektrodenkopf am distalen Ende des Elektrodenkörpers mit
= einem hohlzylinderartigem Gehäuse,
= einer darin mit Hilfe der Zuleitung drehbar und axial verschiebbar gelagerten, elektrisch leitfähigen Welle, die an der Zuleitung in axialer Verlängerung befestigt ist,
= einer schraubendruckfederartigen, starren Einschraubelektrode an der Welle, sowie
= einer zwischen Gehäuse und Welle eingesetzten Kontaktfeder gemäß einem der Ansprüche 1 bis 11 zu deren elektrischer Verbindung, wobei die Kontaktfeder einen Außendurchmesser aufweist, der mit dem Gehäuse elektrisch leitend kontaktierbar ist,
**dadurch gekennzeichnet, dass**
die Welle zwischen zwei Schenkeln der Kontaktfeder gleitend und axial verschiebbar gelagert ist und dass durch die in die elektrische Kontaktierung verbrächte Kontaktfeder radial nach außen gerichtete Federkräfte auf die Schenkel wirken, so dass dadurch die Lage der Schenkel von der vorbestimmten Lage im nicht kontaktierten Zustand entfernt ist und dadurch ein elektrischer Kontakt zwischen Gehäuse und
Welle entsteht.

15. Elektrodenleitung gemäß Anspruch 14, **dadurch gekennzeichnet, dass** das Gehäuse ein Wellenlager aufweist, das die Welle auf einer Gehäusemittelachse lagert, wodurch sich die radial nach außen gerichteten Federkräfte gegenseitig kompensieren.

## Claims

1. A contact spring (1) for electrically contacting an electrode head housing with a shaft mounted inside the electrode head housing, the contact spring having an outer contour with a center line (11), the outer contour being contactable with the housing in an electrically conducting manner, the wire of the contact spring being shaped so that the wire forms at least two legs inside the outer contour, and the positions of the at least two legs in the contacted state of the contact spring being remote from a predetermined position in the non-contacted state due to the action of a force, **characterized in that** the outer contour of the contact spring is formed by four to ten wire windings.

2. The contact spring according to claim 1, **characterized in that** the outer contour of the spring is formed by four to six, and preferably four, wire windings.

3. The contact spring according to claim 1 or 2, **characterized in that** the wire windings are wound around the center line so that the outer contour has a cross-section that is polygonal, preferably triangular, or rounded, preferably circular, oval or of constant thickness.

4. The contact spring according to claims 1 to 3, **characterized in that** no more than two of the wire windings are shaped or bent radially inward from the outer diameter, wherein the diameters of these no more than two windings are smaller than the outside diameter.

5. The contact spring according to claim 4, **characterized in that** the two radially inwardly shaped or bent wire windings form the two legs.

6. The contact spring according to claim 5, **characterized in that** the two legs each form an eyelet.

7. A contact spring according to any one of claims 1 to 6, **characterized in that** the legs, in the non-contacted state, are located laterally offset from the center point axis of the contact spring and, in the contacted state are located aligned with the center line shifted as compared to their predetermined position thereof due to the action of force on the legs.

8. The contact spring according to claim 7, **characterized in that** the legs each have the same distance from the center line of the contact spring.

9. The contact spring according to claim 7 or 8, **characterized in that** each of the legs is located diametrically opposed with respect to the center line of the contact spring.

10. A contact spring according to any one of claims 1 to 6, **characterized in that** the legs, in the non-contacted state, are located aligned with the center line, and the contacted state is achieved by an action of force on the outer contour pointing in the direction of the center line, whereby the legs are remote from the predetermined position.

11. A contact spring according to any one of claims 1 to 10, **characterized by** the height thereof is 0.5 mm to 5 mm, and preferably 0.5 mm.

12. A contact spring according to any one of claims 1 to 11, **characterized in that** the wire is a round or flat wire.

13. A contact spring according to any one of claims 1 to 12, **characterized by** being configured as a torsion spring.

14. An electrode lead for cardiology use, comprising:
- an elongated electrode body made of an electrically insulating material;
- a rotatable electric supply line extending in the electrode body; and
- an electrode head at the distal end of the electrode body, having:
= a hollow cylindrical-shaped housing;
= an electrically conductive shaft, which is mounted therein so as to be rotatable and axially displaceable with the aid of a supply line and which is attached to the supply line in axial extension;
= a helical compression spring-shaped, rigid screw-in electrode on the shaft; and
= a contact spring according to any one of claims 1 to 11 inserted between the housing and the shaft for the electrical connection thereof, wherein the contact spring has an outer diameter that can be contacted with the housing in an electrically conducting manner,
**characterized in that**
the shaft is mounted so as to be slideably and axially displaceable between two legs of the contact spring, and radially outwardly directed spring forces act on the legs due to the contact spring being electrically contacted so that the position of the legs is remote from the predetermined position in the non-contacted state, and thereby an electrical contact is created between the housing and the shaft.

15. The electrode lead according to claim 14, **characterized in that** the housing comprises a shaft bearing, which mounts the shaft on the housing center line, whereby the radially outwardly directed spring forces mutually compensate each other.

## Revendications

1. Ressort de contact (1) pour la mise en contact électrique d'un boîtier de tête d'électrode avec un arbre monté à l'intérieur du boîtier de tête d'électrode, dans lequel le ressort de contact présente un contour extérieur avec un axe médian (11), dans lequel le contour extérieur peut être mis en contact de façon électriquement conductrice avec le boîtier, dans lequel le fil du ressort de contact est conçu de manière à former au moins deux branches à l'intérieur du contour extérieur, dans lequel la position des au moins deux branches dans l'état mis en contact du ressort de contact est éloignée d'une position prédéterminée dans l'état non mis en contact, par l'exercice d'une force, **caractérisé en ce que** le contour extérieur du ressort de contact est constitué de quatre à dix enroulements de fil.

2. Ressort de contact selon la revendication 1, **caractérisé en ce que** son contour extérieur est constitué de quatre à six, de préférence de quatre enroulements de fil.

3. Ressort de contact selon la revendication 1 ou 2, **caractérisé en ce que** les enroulements de fil sont enroulés de telle façon autour de l'axe médian, que le contour extérieur présente une section transversale polygonale, de préférence triangulaire, ou arrondie, de préférence circulaire, ovale ou à épaisseur constante.

4. Ressort de contact selon la revendication 1 à 3, **caractérisé en ce qu'**au maximum deux enroulements de fil sont formés ou coudés radialement vers l'intérieur à partir du diamètre extérieur, dans lequel ces au maximum deux enroulements présentent un diamètre inférieur au diamètre extérieur.

5. Ressort de contact selon la revendication 4, **caractérisé en ce que** les deux enroulements de fil formés ou coudés radialement vers l'intérieur forment les deux branches.

6. Ressort de contact selon la revendication 5, **caractérisé en ce que** les deux branches forment respectivement un oeillet.

7. Ressort de contact selon l'une des revendications 1 à 6, **caractérisé en ce que** dans l'état non mis en contact, les branches sont décalées latéralement par rapport à l'axe médian du ressort de contact, et sont mises en affleurement avec l'axe médian dans l'état mis en contact, par l'exercice d'une force sur les branches, à partir de leur position prédéterminée.

8. Ressort de contact selon la revendication 7, **caractérisé en ce que** les branches présentent respectivement le même espacement par rapport à l'axe médian du ressort de contact.

9. Ressort de contact selon l'une des revendications 7 ou 8, **caractérisé en ce que** chacune des branches est diamétralement opposée par rapport à l'axe médian du ressort de contact.

10. Ressort de contact selon l'une des revendications 1 à 6, **caractérisé en ce que** dans l'état non mis en contact, les branches sont en affleurement par rapport à l'axe médian, et l'état mis en contact est atteint par l'exercice d'une force dirigée en direction de l'axe médian, sur le contour extérieur, éloignant ainsi les branches de la position prédéterminée.

11. Ressort de contact selon l'une des revendications 1 à 10, **caractérisé en ce que** sa hauteur est de 0,5 mm à 5 mm, de préférence de 0,5 mm.

12. Ressort de contact selon l'une des revendications 1 à 11, **caractérisé en ce que** le fil est un fil rond ou plat.

13. Ressort de contact selon l'une des revendications 1 à 12, **caractérisé en ce qu'**il est conçu comme un ressort à branches.

14. Ligne d'électrode pour une application cardiologique, comprenant :
- un corps d'électrode allongé, constitué d'un matériau électriquement isolant,
- une ligne électrique d'arrivée rotative, s'étendant dans le corps d'électrode, et
- une tête d'électrode à l'extrémité distale du corps d'électrode, avec
= un boîtier du genre cylindre creux,
= un arbre électriquement conducteur agencé à l'intérieur de façon axialement déplaçable et rotative à l'aide de la ligne d'arrivée, lequel est fixé à la ligne d'arrivée dans un prolongement axial,
= une électrode vissable fixe sur l'arbre, du genre ressort de pression hélicoïdal,
= un ressort de contact selon l'une des revendications 1 à 11, inséré entre le boîtier et l'arbre pour le raccordement électrique de ceux-ci, le ressort de contact présentant un diamètre extérieur apte à être mis en contact de façon électriquement conductrice avec le boîtier,
**caractérisé en ce que**
l'arbre est monté de façon axialement déplaçable et coulissante entre deux branches du ressort de contact, et **en ce que** la mise en contact électrique du ressort de contact entraîne des forces de ressort dirigées radialement vers l'extérieur, sur les branches, de sorte que la position des branches est éloignée de la position prédéterminée dans l'état non mis en contact, établissant ainsi un contact électrique entre le boîtier et l'arbre.

15. Ligne d'électrode selon la revendication 14, **caractérisé en ce que** le boîtier comporte un palier d'arbre faisant reposer l'arbre sur un axe médian de boîtier, et les forces de ressort dirigées radialement vers l'extérieur se compensant ainsi mutuellement.
